# EUROPEAN PATENT SPECIFICATION

(11) **EP 4 547 154 B1**
(45) Date of publication and mention of the grant of the patent: **27.05.2026**
(21) Application number: 23737972.2
(22) Date of filing: 30.06.2023
(51) Int. Cl.: A61D 1/14, A61M 39/02

(54) **METHANE OXIDATION DEVICE**
METHANOXIDATIONSVORRICHTUNG
DISPOSITIF D'OXYDATION DU MÉTHANE

(30) Priority: 01.07.2022 GB 202209737
(43) Date of publication of application: 07.05.2025
(73) Proprietor: ZELP LTD, London WC2R 0EX (GB)
(72) Inventor: NORRIS, Francisco, London Greater London WC2R 0EX (GB); NORRIS, Patricio, London Greater London WC2R 0EX (GB); GUNDRY, Thomas Trevenen, London Greater London WC2R 0EX (GB)
(74) Representative: Murgitroyd & Company
(86) International application number: PCT/EP2023/068039
(87) International publication number: WO 2024/003369

(56) References cited:
- CN-A- 114 324 772
- DE-C- 172 189
- US-A- 3 039 468
- US-A1- 2010 279 180
- US-A1- 2014 199 225

## Description

### Field of the Invention

The present invention relates to a methane oxidation device for oxidation of methane from a digestive cavity associated with an animal. The invention also relates to an installation gun for installing a methane oxidation device.

### Background to the Invention

Methane is known to be a potent greenhouse gas, having a global warming potential that is considerably higher than that of carbon dioxide. Livestock are known to be a significant source of methane gas, which is released via exhalation and eructation. The methane emission of livestock often has direct economic consequences for livestock producers, who may be subject to taxes based on their carbon footprint. Additionally, with cattle being key contributors to global warming, it is vital to reduce methane emissions from livestock.

A method of reducing the quantity of methane emissions is to capture and oxidise the methane before it is released into the atmosphere. In this process, methane is oxidised with oxygen to produce carbon dioxide and water vapour, reducing the quantity of harmful methane released. The energy demand of this process is high, with a required oxidation temperature of around 500°C. Additionally, recurrent emissions from livestock mean that the need to oxidise this methane arises very frequently.

It can be difficult to capture and oxidise methane after it is emitted from an animal, such as by exhalation, eructation or flatulence. In such cases, correct positioning of the methane oxidation system is paramount to guarantee effective oxidation. As such, there is a benefit in oxidising methane prior to natural emission from the animal.

Another method to reduce methane emissions in cattle is to add dietary supplements to cattle feed to reduce methanogenesis occurring in the rumen. However, such feed additives require regular dosage, possess a low efficiency of methanogenesis reduction and cannot be used with non-cattle feed.

Objects and aspects of the present claimed invention seek to alleviate at least these problems with the prior art.

US 2010/279180 A1 discloses a process for the utilization of the methane produced by enteric fermentation, specifically a process that utilizes methane produced by ruminant animals through enteric fermentation as a source of carbon and/or energy for the directed production of methane-based goods or processes.

DE 172 189 C discloses a mechanically operated device for inserting a trocar into the stomach of a cow. The device comprises a brass tube provided with grooves, a springloaded piston with a handle for tensioning the spring and a trigger.

### Summary of the Invention

According to a first aspect of the invention, as defined in appended claim 1, there is provided a methane oxidation device for oxidation of methane from a digestive cavity associated with an animal, the device comprising a methane oxidation unit for oxidising methane; wherein the methane oxidation unit comprises an inlet portion in fluid communication with the external environment, the inlet portion further configured to be mounted to a hollow member in fluid communication with a digestive cavity associated with an animal, and further when the methane oxidation unit comprises an outlet portion in fluid communication with the external environment, so that methane from a digestive cavity associated with an animal is oxidised in the oxidisation unit prior to release into the external environment.

In this way, there is provided a device for oxidising methane from an animal prior to emission from exhalation, eructation or flatulence. The methane oxidation device is particularly advantageous for use on bovine animals, such as cattle, but use on non-bovine animals, such as sheep and goats, is also envisaged. Difficulties capturing methane emitted from the mouth or snout of an animal can lead to methane escaping into the external environment prior to methane oxidation. As such, a benefit of the present invention is that methane from a digestive cavity of the animal can be oxidised prior to emission to the external environment via the mouth or snout. It is understood that fluid can be gas, liquid and/or a combination of the gas and liquid. Preferably, fluid communication is gaseous communication.

Preferably, the digestive cavity is a rumen of an animal. In this way, a methane oxidation device for oxidising methane from the methane rich rumen of an animal is provided.

The methane oxidation device further comprises; at least one hollow member comprising a first portion and second portion; and a piercing member for piercing a channel into a digestive cavity of an animal, the piercing member located at the first portion of the at least one hollow member, wherein the methane oxidation unit is located at the second portion of the at least one hollow member; wherein the first portion of the at least one hollow member is in fluid communication with a digestive cavity associated with an animal.

In some embodiments, the at least one hollow member consists of one hollow member. In other embodiments, the at least one hollow member comprises a plurality of hollow members. In embodiments comprising a plurality of hollow members, all hollow members may be in fluid communication with the methane oxidation unit. Alternatively, the methane oxidation unit comprises a plurality of methane oxidation units and one or more hollow members of the plurality of hollow members is in fluid communication with each methane oxidation unit of the plurality of methane oxidation units.

Preferably, the methane oxidation unit is located between the at least one hollow member and the external environment. In this way, an efficient fluid pathway from the digestive cavity of an animal to the external environment is provided.

Preferably, the methane oxidation unit comprises at least one catalytic material. Preferably, the at least one catalytic material is located between the inlet portion and the outlet portion and at least a portion of the at least one catalytic material is arranged in a permeable configuration so as to allow fluid flow from the inlet portion to the outlet portion. It is understood that a permeable configuration is one that allows air to flow from the inlet portion to the outlet portion. For example, the catalytic material is permeable and/or the arrangement of the catalytic material allows fluid flow from the inlet portion to the outlet portion. For example, the catalytic material may be arranged in a grid or mesh configuration and/or comprises a plurality of even or unevenly distributed apertures or other vents. Preferably, the outlet portion is configured to inhibit liquid flow from the external environment into the methane oxidation device. It is advantageous to prevent rain and other contaminants entering the methane oxidation unit from the external environment.

Preferably, the methane oxidation device comprises a retaining member configured to removably retain the methane oxidation unit to an animal. Preferably, the retaining member is located at the first portion of the at least one hollow member and comprises a collapsible portion wherein retraction of the methane oxidation device through a channel into a cavity of an animal draws the first portion towards the second portion for collapsing the collapsible portion, the collapsible portion configured to splay outwardly with respect to a longitudinal axis of the at least one hollow member as it collapses.

Alternatively, the retaining member is located at the second portion of the at least one hollow member. Alternatively, the retaining member is located on the methane oxidation unit. Alternatively, the retaining member is located on at least one of; the first portion; the second portion; and the oxidation unit. In some embodiments, the retaining member comprises at least one piercing member configured to pierce the skin of an animal. In some embodiments, the retaining member comprises an adhesive configured to adhere to a surface of an animal. Preferably, the surface is the skin of an animal. In some embodiments, the retaining member comprises positioning means to position the methane oxidation device to an animal. For example, the positioning means may comprise straps.

Preferably, the at least one hollow member comprises a valve located in the second portion and configured to inhibit fluid flow exiting the second portion of the at least one hollow member. Preferably, the valve is a one-way valve. In this way, the fluid flow from the digestive cavity of an animal cannot bypass the methane oxidation unit by exiting to the external environment from the second portion prior to oxidation. Preferably, the valve is configured to allow fluid flow when the force of fluid experienced by the valve is above a predetermined value. For example, the valve thickness, valve material or other valve characteristic is selected to fit the desired predetermined value. In this way, when fluid pressure is such that the device may be dislocated or removed from the animal, fluid may escape to the external environment, bypassing the methane oxidation unit and preventing device expulsion.

Preferably, the outlet portion comprises at least one aperture located on an external surface of the methane oxidation unit. More preferably, the outlet portion comprises a plurality of vents located on an external surface of the methane oxidation unit. **In** this way, the products of the methane oxidation reaction, namely carbon dioxide and water vapour, can enter the external environment after oxidation.

Preferably, the methane oxidation device comprises a sensor. Preferably, the sensor is located in the methane oxidation unit. For example, the sensor may comprise a methane sensor, carbon dioxide sensor, inertia sensor, pressure sensor, flow rate sensor and/or temperature sensor. In this way, at least one characteristic of the digestive cavity emission characteristics, oxidation conditions and/or flow characteristics can be detected and monitored by the user, such as the efficacy of the methane conversion.

Preferably, the methane oxidation device comprises a data transfer unit configured to transfer data from the sensor to an external device. Preferably, the data transfer unit is configured to wirelessly transfer data from the sensor to an external device. In this way, data regarding the digestive cavity emission characteristics, oxidation conditions and/or flow characteristics can be monitored by the user from an external location.

Preferably, the at least one hollow member comprises a flow regulator configured to inhibit fluid flow into the methane oxidation unit when the sensor detects a characteristic of the device is at a predetermined threshold. Alternatively, the flow regulator is located in the methane oxidation unit. For example, the characteristic may be a fluid flow characteristic such as the methane concentration level, or a device characteristic such as the temperature within the methane oxidation unit or the temperature within the at least one hollow member. In some embodiments, the sensor detects a plurality of flow characteristics. Preferably, the predetermined threshold is a predetermined maximum temperature within the methane oxidation unit. In this way, the methane oxidation device comprises a temperature dependant regulator. In some embodiments, the flow regulator comprises a spring.

Oxidation of methane is an exothermic reaction. As such, there is benefit in inhibiting methane flow into the methane oxidation unit when the sensor detects that the temperature within the methane oxidation unit reaches a predetermined maximum temperature. By inhibiting methane flow into the methane oxidation unit, temperature increase within the methane oxidation unit is also inhibited, reducing the risk of burns or other injury to the user or an animal in contact with the methane oxidation unit.

Preferably, the methane oxidation unit comprises a thermal barrier configured to inhibit heat transfer from the methane oxidation unit to the external environment. In this way, heating of the external environment is slowed or inhibited to reduce risk of burns and other injuries to a user or an animal to which the methane oxidation device is mounted. In some embodiments, the methane oxidation unit comprises an insulation unit for insulating the methane oxidation unit to inhibit heat loss to the external environment. In this way, a more efficient device is provided and there is greater ease in maintaining the energy demand for the oxidation process.

Preferably, the methane oxidation device comprises an external abutting portion comprising a thermal barrier configured to inhibit heat transfer from the methane oxidation unit to the external abutting portion. Preferably, the methane oxidation unit and the first portion of the at least one hollow member comprises an external abutting portion. The external abutting portion may be configured to abut the surface of an animal. In this way, when the methane oxidation device is retained on an animal, heat from the methane oxidation unit is inhibited from burning or causing discomfort to the animal due to the temperature of the methane oxidation unit.

Preferably, the methane oxidation unit comprises a thermal store configured to retain thermal energy from a methane oxidation reaction in the methane oxidation unit. Preferably, the thermal store comprises a thermal energy retaining material. For example, the thermal store may be configured in a honeycomb or as a plate. In this way, the temperature within the methane oxidation unit can be maintained over a greater period, increasing the duration in which methane oxidation reactions occurring within the methane oxidation unit are facilitated.

In some embodiments, the methane oxidation device comprises a heat source. Preferably, the heat source is located in the methane oxidation unit. In this way, a more efficient device is provided and there is greater ease in maintaining the energy demand for the oxidation process. In some embodiments, the heat source comprises a resistor.

As the flow of methane from the digestive cavity of an animal is predictable and due to the exothermic nature of the methane oxidation reaction, the device may not require an internal or external power source or heat source. A standard rumen of a cow comprises methane in high purities. As such, the oxidation unit is supplied by a methane rich fluid stream, promoting continuous oxidation.

Preferably, the methane oxidation unit is dome-shaped. Preferably, the at least one hollow member is straight.

Preferably, the diameter of the at least one hollow member is from 0.5 mm to 40 mm. More, preferably, the diameter of the at least one hollow member is from 0.5 mm to 20 mm. More preferably, the diameter of the at least one hollow member is from 2 mm to 10 mm.

Preferably, the length of the portion of the at least one hollow member located between the methane oxidation unit and the piercing member is from 30 mm to 70 mm. More preferably, the length of the portion of the at least one hollow member located between the methane oxidation unit and the piercing member is from 30 mm to 40 mm. **In** this way, the depth of insertion of the methane oxidation device into the digestive cavity associated with the animal is adequate to create a fluid pathway between the digestive cavity associated with an animal and the external environment.

Preferably, the first portion of at least one hollow member is in fluid communication with the external environment via a permeable mesh. Preferably, the permeable mesh extends about the circumference of the first portion of at least one hollow member. In this way, large debris and matter from the digestive cavity of the animal is inhibited from entering the at least one hollow member and blocking the passage of fluid. In some embodiments, the permeable mesh abuts the piercing member.

Preferably, the at least one hollow member comprises an unclogging member configured to unclog accumulated matter from at least a portion of the at least one hollow member. **It** is understood that unclogging comprises any cleaning, flushing or clearing action performed by the unclogging member to remove some or all debris and other accumulated matter from the at least one hollow member. Preferably, the unclogging member is configured to unclog accumulated matter from the permeable mesh. Preferably, the unclogging member comprises a key complementary to the permeable mesh.

Preferably, the unclogging member is actioned when the pressure differential between the digestive cavity of an animal and the at least one hollow member falls to a predetermined value. In this way, the unclogging member moves towards and engages with the permeable mesh as fluid flows back into the cavity from the at least one hollow member, thereby unclogging the permeable mesh. Cattle regularly exhibit rumen contraction events which cause the pressure differential between the digestive cavity of an animal and the at least one hollow member to fall. These naturally occurring events are thereby exploited by the unclogging member to clean the at least one hollow member. As such, the unclogging member is actioned periodically over time and no dedicated driving force is required to action the unclogging member.

Alternatively or additionally, the unclogging member comprises a piston. Preferably, the piston is configured in transition fit with the at least one hollow member. Preferably, the piston is actioned when the pressure differential between the digestive cavity of an animal and the at least one hollow member falls to a predetermined value. In this way, the piston can periodically clear debris and other accumulated matter from the hollow cavity of the at least one hollow member, including debris located on the internal walls and the permeable mesh.

Preferably, the methane oxidation device comprises a methane dilution unit configured to dilute the concentration of methane. Preferably, the methane dilution unit is located in the methane oxidation device. Preferably, the methane oxidation unit comprises at least one dilution inlet configured to allow fluid from the external environment to enter the methane oxidation unit. In this way, the methane rich fluid flow from the digestive cavity of the animal is mixed with low-methane air from the external environment prior to catalysis. The methane dilution unit is beneficial in preventing overheating of the methane oxidation unit.

Preferably, the methane oxidation unit comprises a cooling unit configured to lower the temperature within the methane oxidation unit. Preferably, the cooling unit comprises at least one cooling inlet configured to allow fluid from the external environment to enter the methane oxidation unit. In this way, air from the external environment can mix with the oxidised air flow from the methane oxidation unit allowing heat transfer from the oxidised fluid flow to the air from the external environment. As such, the products of the exothermic oxidation reaction are cooled prior to emission from the outlet portion.

Preferably, the at least one hollow member comprises a buoyant member configured to float inside the digestive cavity associated with the animal. More preferably, the buoyant member is configured to float on the surface of the liquid within the digestive cavity associated with an animal. Preferably, the buoyant member is located on the first portion of the at least one hollow member. In this way, the first portion of the at least one hollow member is configured to remain buoyant and positioned within any gas pocket of the digestive cavity of an animal. As such, the methane oxidation device can oxidise methane from any gas pocket and will not be submerged in any liquid or other matter in the digestive cavity, In some embodiments, the buoyant member comprises a flexible elongate portion configured to extend the fluid path of the at least one hollow member. In this way, the buoyant member allows the at least one hollow member to extend further within the digestive cavity associated with an animal, reaching any distal gas pockets in the cavity.

The present invention provides a portable device with a low resource demand. Given the large number of animals present in a herd, such as a herd of cattle, an inexpensive and replaceable device is particularly advantageous. Further, the age, size and type of animal which the device can be used on is not particularly limited, and there is a reduced requirement to select the size of the device to better fit each individual animal.

Preferably, the at least one hollow member comprises an inlet valve located in the second portion and configured to permit fluid flow entering the second portion of the at least one hollow member from the external environment. Preferably, the valve is a one-way valve. In this way, the fluid flow from the external environment can enter the digestive cavity of an animal. As such, with every rumen contraction, the methane exiting the digestive cavity of an animal can mix with air and oxygen from the external environment as it travels through the hollow member prior to oxidation. In this way, the methane content of the fluid exiting the digestive cavity of an animal is diluted prior to oxidation.

In some embodiments, the valve inhibiting fluid flow exiting the second portion of the at least one hollow member comprises the valve permitting fluid flow from the external environment into the second portion. In this way, the valve is a two-way valve.

In some embodiments, the at least one hollow member comprises an outlet valve located in the second portion and configured to permit fluid flow exiting the second portion of the at least one hollow member into the external environment. In this way, the valve is a one-way valve that permits fluid exiting the rumen to leave the methane oxidation device, while inhibiting fluid from the external environment from entering the methane oxidation device.

As such, the methane oxidation device may comprise; a one-way inlet valve permitting fluid from the external environment to mix with the fluid exiting the rumen; a one-way outlet valve permitting fluid exiting the rumen to leave the methane oxidation device, while inhibiting fluid from the external environment from entering the methane oxidation device; and/or a two-way valve permitting fluid to enter the methane oxidation device from the external environment and permitting fluid inside the methane oxidation device to exit the methane oxidation device.

In some embodiments, the self-sealing valve comprises a floating member configured to occlude the opening of the at least one hollow member in the second end. Preferably, the floating member comprises a ball. Preferably, the floating member is configured to move between the opening of the at least one hollow member in the second end and a second position within the at least one hollow member. In this way, when a large volume of fluid from the rumen passes through the at least one hollow member and attempts to exit the open end of the at least one hollow member, the floating member is pushed towards the open end of the at least one hollow member, thereby occluding the open end and preventing the fluid from exiting the at least one hollow member.

Preferably, the methane oxidation device further comprises a filter configured to permit gas flow through the filter but inhibiting liquid flow through the filter. Preferably, the permeable mesh comprises the filter. Alternatively, the filter is located in the first end of the at least one hollow member. Preferably, the filter is located across the entire width of the at least one hollow member.

In this way, methane may enter the at least one hollow member, and subsequently the methane oxidation device, without unwanted liquids from the rumen of the animal entering the at least one hollow member. Advantageously, efficiency of the methane oxidation unit is improved and the reduction in contaminants improves the lifespan of the methane oxidation device.

Preferably, the filter comprises ceramic or Polytetrafluoroethylene (PTFE).

Preferably, the retaining member is adjustable. In this way, the retaining member can be adjusted to fit the dimensions of the animal to which the methane oxidation device is to be fixed. Preferably, the retaining member comprises a resilient member configured to adjust at least one dimension of the retaining member. For example, when the retaining member comprises a contractible portion, preferably, the contractible portion is coupled to a resilient member such that the resilient member is configured to control the degree of contraction by abutting a portion of an animal. Preferably, the resilient member biases the retaining member to the minimum level of contraction. Namely, without the presence of an animal, the retaining member is biased to the minimum level of contraction. Therefore, when the methane oxidation device is attached to a large animal, the resilient member abuts a portion of the animal such that the degree of contraction of the contractible portion is greater to accommodate the animal. In some embodiments, the resilient member is a spring.

Preferably, the methane oxidation unit is removably retainable on at least one hollow member. In this way, the user may remove and replace the methane oxidation unit, such as for maintenance or replacement due to damage or fault.

Preferably, the methane oxidation device further comprises a methane measuring unit configured to measure at least one characteristic of the air inside or exiting at least one hollow member. For example, the at least one characteristic may be the concentration of methane in the air. Preferably, the methane measuring unit comprises at least one sensor. Preferably, the methane measuring unit is removably retainable on at least one hollow member. In this way, the user may remove and replace the methane measuring unit, such as for maintenance or replacement due to damage or fault. The user may therefore chose whether to attach the methane measuring unit or the methane oxidation unit to the at least one hollow member. The user may swap between each unit depending on the desired 'mode' of the methane oxidation device, namely the methane sensing mode and the methane oxidation mode. In select embodiments, the at least one hollow member is configured to house both a methane oxidation unit and a methane measuring unit.

Preferably, the methane oxidation unit comprises an air flow regulator configured to regulate the flow of air within the at least one hollow member. As the flow of air entering the at least one hollow member from the rumen of the animal will vary in both volume and flow-rate, a flow regulator improves regulation of the air flow. In this way, the methane oxidation unit efficiency is improved, as a regular flow of air enters the methane oxidation unit to be oxidised. Overloading of the methane oxidation unit is therefore reduced and the air kinetics are more predictable. Preferably, the air flow regulator is located within the at least one hollow member. In some embodiments, the air flow regulator is located in the first portion of the at least one hollow member. In this way, pressure within the at least one hollow member does not build up when a high volume of air attempts to pass the air flow regulator.

According to a second aspect of the present invention, as defined in appended claim 15, there is provided an installation gun for installing a methane oxidation device according to the first aspect of the present invention; the installation gun comprising; a methane oxidation device holder configured to hold a methane oxidation device according to the first aspect of the present invention; an insertion unit configured to insert a portion of a methane oxidation device held on the methane oxidation device holder into a digestive cavity associated with an animal; an actuation member configured for user actuation of the insertion unit; and an ignition member configured to produce a spark.

In this way, the installation gun can be used to provide the force required for the piercing member of the methane oxidation device to pierce the skin of an animal. Upon actuation of the installation gun, the insertion unit then inserts the at least one hollow member into a digestive cavity of an animal. The methane oxidation unit is then in fluid communication with the digestive cavity. The ignition member can then produce a spark to ignite the methane rich fluid from the digestive cavity which is located within the methane oxidation unit. Such a spark heats the thermal store of the methane oxidation unit, flares the methane and thereby facilitates methane oxidation within the oxidation unit.

Preferably, the methane oxidation device holder comprises a rod configured to be housed within the at least one hollow member. Preferably, the piercing member is configured to be removably retained on a first end of the rod. In this way, the insertion force applied to the rod by the user is transferred to the piercing member and the installation gun can thereafter be removed from the methane oxidation device and the digestive cavity of the animal.

Preferably, the actuation member comprises a handle member comprising a trigger configured for actuation by a users' hand. Such an actuation member allows the user to apply the necessary force to the skin of the animal to insert the methane oxidation device into the digestive cavity.

Preferably, the insertion unit is configured to actuate the retaining member. Preferably, the installation gun comprises a fan. Such a fan aids methane ignition and dilution. In some embodiments, the installation gun comprises a power source configured to aid ignition of the ignition member. For example, the installation gun may comprise batteries or may be configured to receive power from a mains power supply.

### Detailed Description

Embodiments of the present invention will now be described by way of example only and with reference to the accompanying drawings, in which:
Figure 1 depicts a methane oxidation device of the first aspect of the present invention retained on a cow for oxidation of methane from the rumen of the cow;
Figure 2A depicts a view of the exterior of the methane oxidation unit of the methane oxidation device of Figure 1;
Figure 2B depicts a view of the contractible portion of the retaining member of the methane oxidation device of Figure 1;
Figure 3 depicts the methane oxidation device of Figure 1 in communication with an installation gun of the second aspect of the present invention; and
Figure 4 depicts a further view of the methane oxidation device of Figure 1 with the retaining member in a collapsible position, the fluid flow paths of the methane oxidation device illustrated.

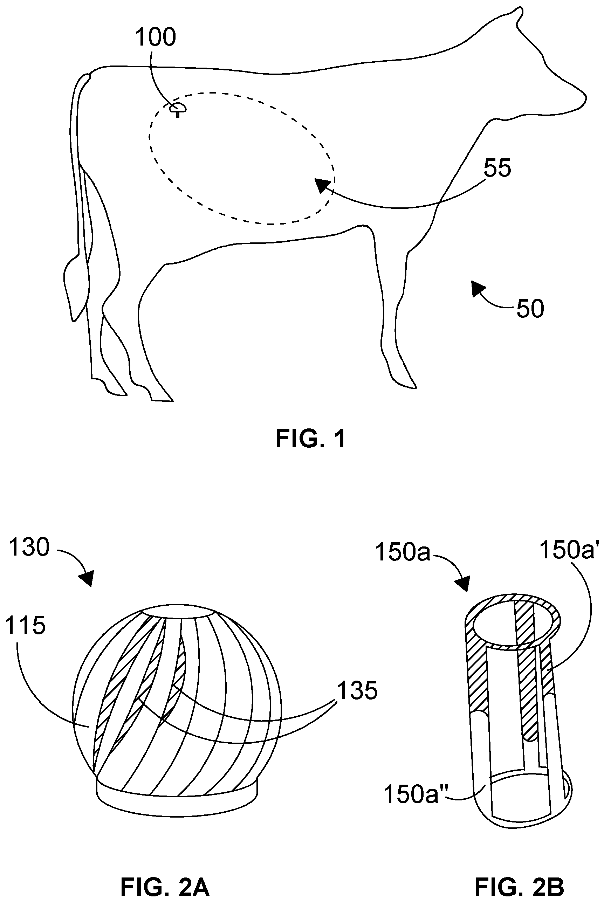

With reference to Figures 1-4, there is illustrated a methane oxidation device 100 retained on a cow 50. As illustrated on Figure 1, the device 100 is affixed to the skin of the cow 50 above a digestive cavity of the cow 50. In this embodiment, the digestive cavity is the rumen 55 and the device 100 is configured to oxidise methane from the rumen 55.

The device 100 comprises a hollow member 105 comprising a first portion 105a and a second portion 105b. The hollow member 105 is straight and cylindrical and has a constant cross section along its length. In this embodiment, the hollow member 105 comprises silicone. The device 100 further comprises a piercing member 110 located at the first portion 105. The piercing member 110 comprises a tip sufficiently sharp to pierce a channel from the skin, through rumen lining 60 and into the rumen 55 of the cow 50. The piercing member 110 extends along a longitudinal axis L of the hollow member 105. The piercing member creates a straight channel 60a through the rumen lining 60. **In** this way, the hollow member 105 and piercing member 110 form a hollow needle.

The first portion 105a comprises a permeable mesh 125 which extends about the circumference of the first portion 105a and abuts the piercing member 110. When the device 100 is affixed to the cow 50, as illustrated in Figures 1-4, the mesh 125 allows fluid from the rumen 55 to enter and traverse the hollow member 105. The mesh 125 also allows fluid to exit the hollow member 105 and return to the rumen 55. In this way, the first portion 105a of the hollow member 105 is in fluid communication with the rumen 55.

The device 100 further comprises a dome-shaped methane oxidation unit 115 for oxidising methane located at the second portion 105b of the hollow member 105. The methane oxidation unit 115 is located between the hollow member 105 and an external environment 95 of the device 100. The second portion 105b is located in a central aperture of the methane oxidation unit 115 and does not extend beyond the methane oxidation unit 115, such that a compact device is provided.

The methane oxidation unit 115 comprises an inlet portion 120 comprising an aperture in fluid communication with the first portion 105a of the hollow member 105. The inlet portion 120 is located adjacent the second portion 105b of the hollow member 105 and extends about the circumference of the hollow member 105. The second portion 105b of the hollow member comprises a complementary aperture about its circumference. The inlet portion 120 permits fluid flow from the methane oxidation unit 115 to hollow member 105 and vice versa. As the first portion 105a is in fluid communication with the rumen 55, fluid from the rumen 55 can traverse the hollow member 105 and enter the methane oxidation unit 115 via the inlet portion 120.

The methane oxidation device 115 further comprises an outlet potion 130 in fluid communication with the external environment 95. As depicted on Figure 2A, the outlet portion 130 comprises a plurality of vents 135 located on an external surface of the methane oxidation unit 115, each vent extending about the circumference of the dome-shaped methane oxidation unit 115 such that a helical chimney is provided. The plurality of vents 135 permit fluid flow from the methane oxidation unit 115 to the external environment 95 and vice versa. The plurality of vents 135 inhibit rainfall from entering the methane oxidation unit 115. As such, the device 100 is configured such that fluid can flow from the rumen 55, through the hollow member 105, into the methane oxidation device 115 and into the external environment 95. As such, methane from a rumen 55 of the cow 50 is oxidised in the oxidisation unit prior to release into the external environment 95.

To fit the device to the cow 50 for oxidation of methane from the rumen 50, the device 100 can be placed on the skin of the cow 50 above wherein the rumen 55 is located. For ease of application, the piercing member 110 is placed on the skin such that the longitudinal axis L of the hollow member 105 is about perpendicular to the surface of the skin. Force can then be applied on the device 100 such that the piercing member 110 pierces the skin and a channel 60a is formed in the rumen lining 60.

A passage is thereby created to the upper part of the rumen 50. Due to the difference in pressure between the rumen 55 and the external environment 95, the gas in the rumen escapes through the hollow member 105 to the external environment 95 without any additional input force or aid from the device 100. The user applies a force until the external abutting portion 165 abuts the skin and the device is considered fully inserted into the cow 50. In the rumen 55 of a cow 50, methane exists in purities of around 270,000 per parts million (ppm).

The methane oxidation unit 115 comprises a catalytic material 140 located between the inlet portion 120 and the plurality of vents 135. The catalytic material 140 is in a honeycomb configuration such that the catalytic material 140 is permeable and an adequate surface area of the catalytic material 140 is provided. Methane can therefore flow from the inlet portion 120, contact the catalytic material 140 which catalyses oxidation of the methane, and the by-products of the oxidation reaction can exit the methane oxidation unit 115 via the plurality of vents 135.

The methane oxidation unit 115 further comprises a thermal store 185 configured to retain thermal energy from the methane oxidation reactions occurring within the methane oxidation unit. The thermal store 185 is a metallic plate located between the second portion 105b of the hollow member 105 and the catalytic material 140.

The hollow member 105 comprises a one-way self-sealing valve 145 located in the second portion 105b and configured to inhibit fluid flow exiting the second portion 105b of the hollow member 105. The valve 145 is located adjacent the inlet portion 120 of the methane oxidation unit 115 but distal the first portion 105a and extends across the diameter of the hollow member 105, blocking fluid flow through the end of the second portion 105b. In this way, the fluid flow from the rumen 55 cannot bypass the methane oxidation unit 115 by entering the external environment 95 prior to oxidation. The valve 145 is configured to allow fluid flow into the external environment 95 via the second portion 105b when the force of fluid experienced by the valve 145 is above a predetermined value.

The hollow member 105 further comprises a flow regulator 180 configured to inhibit fluid flow entering the methane oxidation unit 115. The flow regulator 180 is located in the second portion 105b of the hollow member 105 and comprises a spring 180a fixed adjacent the valve 145 and configured to extend and contract along the longitudinal axis L. In an extended configuration, the spring 180a passes over the aperture of the inlet portion 120. The flow regulator 180 further comprises a blocking member 180b configured to block fluid flow along the hollow member 105. In an extended configuration, the spring 180a extends such that the blocking member 180b blocks fluid flow from the first portion 105a of the hollow member 105 into the inlet portion 210 of the methane oxidation unit 115.

The methane oxidation unit 115 comprises a temperature sensor, and when the temperature in the methane oxidation unit 115 reaches a predetermined threshold, the spring 180a of the flow regulator 180 is configured to extend such that the blocking member 180b blocks fluid flow into the inlet portion 210.

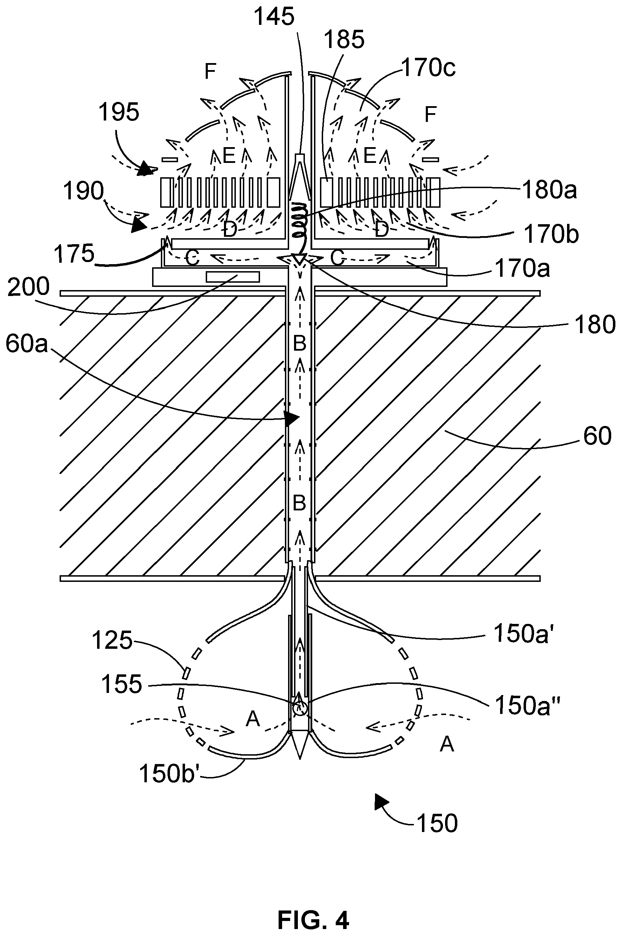

The device 100 further comprises a retaining member 150 configured to removably retain the methane oxidation unit 115 to the cow 50. The retaining member 150 is located at the first portion 105a of the hollow member 105 and comprises a contractible portion 150a, as illustrated in Figure 2B, comprising rigid plastic. The contractible portion 150a comprises an upper part 150a' and a lower part 150a". Retraction of the device 100 through the channel 60a into the rumen 50 draws the lower part 150a"towards the upper part 150a' for contraction the contractible portion 150a. The contractible portion 150a is configured to contract along the longitudinal axis L of the hollow member 105 as it contracts, as illustrated in Figure 4. The contractible portion 150a contracts to a plurality of predetermined lengths. In this way, a ratchet member is provided.

The retaining member 150 further comprises an outwardly splaying portion 150b comprising silicone. The splaying portion 150b comprises a rivet bulb 150b' and the permeable mesh 125. The contractible portion 150a comprises three apertures extending parallel to the longitudinal axis L, thereby allowing fluid flow across the contractible portion 150a. As such, fluid from the rumen 55 can pass through both the permeable mesh 125 of the splaying member 150b and the contractible portion 150a and enter the hollow member 105.

The contractible portion 150a has a greater rigidity than the splaying portion 150b, with the splaying portion 150b have a greater flexibility than the contractible portion 150a. The splaying portion 150b is located between the external environment 55 and the contractible portion 150a, such that motion of the splaying portion 150b is not inhibited by the contractible portion 150a.

The splaying portion 150b is configured to splay outwardly with respect to the longitudinal axis L of the hollow member 105 when the device 100 is retracted back through the channel 60a into the rumen 50, In this way, when the installation gun is removed from the device 100, the contractible portion 150a contracts and the splaying portion 150b splays outwardly such that the retaining member 150 is retained within the rumen 50, as illustrated in Figure 4. The splaying portion 150b inhibits the device exiting the rumen 50 and being removed from the cow 50.

To remove the device 100 from the cow 50, the reverse process can be undertaken. A force towards the rumen 50 can be provided, thereby extending the contractible portion 150a to an uncontracted configuration and further causing the splaying portion 150b to return to an unsplayed configuration, thereby allowing removal of the device through the channel 60a.

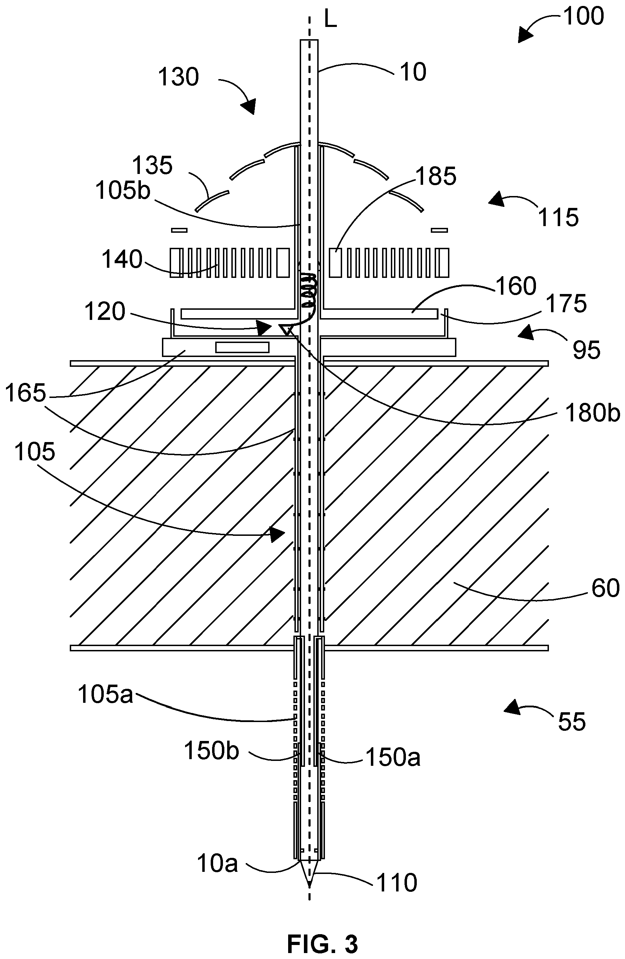

An installation gun in accordance with the second aspect of the invention can be used to install the device 100 onto the cow 50. The installation gun comprises a methane oxidation device holder comprising an elongate rod 10. The piercing member 110 is configured to be removably retained on a first end 10a of the rod 10, as illustrated in Figure 3. After installation, the rod 10 can be removed from the hollow member 105, thereby disconnecting from the piercing member 110.

The first portion 105a further comprises a buoyant member 155. The buoyant member 155 aids in maintaining the first portion's 105a position within a gas pocket of the rumen 55. The buoyant member 155 inhibits sinking of the first portion 105a into liquid or other rumen matter, thereby maintaining fluid communication between the gas pocket of the rumen 55 and the external environment 95.

The device 100 comprises an external abutting portion 165 comprising a thermal barrier 160 configured to inhibit heat transfer from the methane oxidation unit 115 to the external abutting portion 165. The thermal barrier 160 comprises an insulation manifold plate. The external abutting portion 165 comprises a silicone sheet and is located on the skin and rumen lining 60 contacting surfaces of the methane oxidation unit 115 and the hollow member 105.

As such, the external abutting portion 165 inhibits the skin of the cow 50 and the rumen lining 60 next to the channel 60a from burns or other discomfort due to the temperature of the oxidation reaction in the methane oxidation unit 115. In this way, only the external abutting portion 165 of the device 100 is in contact with the surface of the cow 50, improving safety and comfort for the cow 50.

The methane oxidation unit 115 comprises an inlet chamber 170a, a lower chamber 170b and an upper chamber 170c.

The inlet chamber 170a is located adjacent the inlet portion 120. Fluid entering the methane oxidation unit 115 via the inlet portion 120 first passes through the inlet chamber 170a. The upper surface of the inlet chamber 170a comprises the thermal barrier 160. The inlet chamber 170a is in fluid communication with the lower chamber 170b via a plurality of apertures 175 located in the thermal barrier 160. The plurality of apertures 175 are located distal the hollow member 105 and adjacent an exterior surface of the methane oxidation unit 115 such that fluid entering the methane oxidation unit 115 via the inlet portion 120 traverses the majority of the length of the inlet chamber 170a prior to entering the lower chamber 170b.

In this way, excess heat from the oxidation reaction occurring in the lower chamber 170b and upper chamber 170b may be transferred to the fluid in the inlet chamber 170a. The heat energy is therefore partially dissipated prior to heating of the external abutting portion 165. The lower chamber 170b and upper chamber 170c are in fluid communication and are separated by the permeable catalytic material 140.

As depicted on Figure 4, due to the pressure differential between the rumen 55 and the external environment 95, methane from the rumen 55 enters the hollow member 105 via the permeable mesh 125 in Direction A. The methane to be oxidised then traverses the hollow tube 105 in Direction B. The methane to be oxidised passes through the inlet portion 120 and traverses the length of the inlet chamber 170a in Direction C. The methane to be oxidised enters the lower chamber 170b via the plurality of apertures 175 and passes through the honeycomb of catalytic material 140 in Direction D. The catalytic material 140 catalyses oxidation of the methane occurring in the lower chamber 170b and upper chamber 170a. The by-products of the oxidation reaction and any un-oxidised methane enter the upper chamber 170c in Direction E and can exit the methane oxidation unit 115 via the plurality of vents 135 in Direction F.

The inlet chamber 170a comprises a methane sensor 200 configured to monitor the quantity of methane entering the inlet chamber 170a and consequently the quantity of methane undergoing oxidation in the oxidation unit 115.

The lower chamber 170b comprises a methane dilution unit comprising a plurality of dilution inlets 190 located about the circumference of the methane oxidation unit 115. Air from the external environment can enter the lower chamber 170b via the dilution inlets 190 and mix with the methane to be oxidised moving in Direction D. Air from the external environment is likely to have a far lower methane concentration than the fluid flow from the rumen 55. As such, the concentration of the methane in the fluid flowing toward the catalytic material 140 in Direction D is reduced.

The upper chamber 170c comprises a cooling unit comprising a plurality of cooling inlets 195 located about the circumference of the methane oxidation unit 115. Air from the external environment can enter the upper chamber 170c via the cooling inlets 195 and mix with the products of the oxidation reaction from the catalytic material 140 moving in Direction E. Air from the external environment is likely to have a far lower temperature than the fluid flow from the rumen 55. As such, the thermal energy of post-oxidation fluid is transferred to the air from the external environment, and the temperature of the fluid flowing toward the plurality of vents 135 in Direction E is reduced.

The hollow member 105 comprises an unclogging member (not pictured) configured to unclog accumulated matter from the permeable mesh 125. The unclogging member comprises a silicone key complementary to the permeable mesh 125 and is actioned periodically over time when the pressure differential between the rumen 55 and the hollow member 105 falls below a predetermined value.

The methane oxidation unit 115 further comprises a Near Field Communication (NFC) sensor located in the methane oxidation unit 115. The NFC-sensor is an integrated temperature sensor to monitor the temperature of and/or around the catalyst, such as the temperature in the lower chamber 170b and/or upper chamber 170c. The device 100 further comprises a data transfer unit configured to wirelessly transfer data from the sensor to an external device. The temperature data from the sensor can be averaged to provide a daily measurement. Over the lifetime of the device 100, the device 100 can be scanned by an external NFC unit configured to receive data from the data transfer unit. The data can then be analysed to, for example, monitor the amount of methane being oxidised in the exothermic process so as to validate the emissions being reduced for transparency, traceability and carbon offsetting monitoring.

The invention is not limited to the specific examples or structures illustrated, a greater number of components than are illustrated in the figures could be used, for example.

## Claims

1. A methane oxidation device (100) for oxidation of methane from a digestive cavity (55) associated with an animal (50), the device (100) comprising:
at least one hollow member (105) comprising a first portion (105a) and second portion (105b);
a piercing member (110) configured to pierce a channel (60a) into said digestive cavity (55), the piercing member (110) being located at the first portion (105a) of the at least one hollow member (105) such that the first portion (105a) is configured to be arranged in fluid communication with said digestive cavity (55); and
a methane oxidation unit (115) for oxidising methane and configured to be located at the second portion (105b) of the at least one hollow member (105);
wherein the methane oxidation unit (115) comprises:
an inlet portion (120) in fluid communication with the external environment (95) and mounted to the at least one hollow member (105); and
an outlet portion (130) in fluid communication with the external environment (95),
so that methane from said digestive cavity (55) associated with said animal (50) is oxidised in the oxidisation unit (115) prior to release into the external environment (95).

2. The methane oxidation device (100) of claim 1, wherein the methane oxidation unit (115) is located between the at least one hollow member (105) and the external environment (95).

3. The methane oxidation device (100) of claim 1 or claim 2, wherein the methane oxidation unit (115) comprises at least one catalytic material (140); and preferably
wherein the at least one catalytic material (140) is located between the inlet portion (120) and the outlet portion (130) and at least a portion of the at least one catalytic material (140) is arranged in a permeable configuration so as to allow fluid flow from the inlet portion (120) to the outlet portion (130).

4. The methane oxidation device (100) of any preceding claim, wherein the methane oxidation device (115) comprises a retaining member (150) configured to removably retain the methane oxidation unit (115) to said animal (50); and preferably
wherein the retaining member (150) is located at the first portion (105a) of the at least one hollow member (105) and comprises a collapsible portion (150b) wherein retraction of the methane oxidation device (115) through the channel (60a) into the cavity (55) of the animal (50) draws the first portion (105a) towards the second portion (105b) for collapsing the collapsible portion (150b), the collapsible portion (150b) configured to splay outwardly with respect to a longitudinal axis (L) of the at least one hollow member (105) as it collapses.

5. The methane oxidation device (100) of any preceding claim, wherein the at least one hollow member (105) comprises a valve (145) located in the second portion (105b) and configured to inhibit fluid flow exiting the second portion (105b) of the at least one hollow member (105).

6. The methane oxidation device (100) of any preceding claim, wherein the outlet portion (130) comprises a plurality of vents (135) located on an external surface of the methane oxidation unit (115).

7. The methane oxidation device (100) of any preceding claim, wherein the methane oxidation device (100) comprises a sensor (200); and preferably
wherein the methane oxidation device (100) comprises a data transfer unit configured to transfer data from the sensor (200) to an external device; and/or preferably
wherein the at least one hollow member (105) comprises a flow regulator (180) configured to inhibit fluid flow into the methane oxidation unit (115) when the sensor (200) detects a characteristic of the device (100) is at a predetermined threshold.

8. The methane oxidation device (100) of any preceding claim, wherein the methane oxidation unit (115) comprises a thermal barrier (160) configured to inhibit heat transfer from the methane oxidation unit (115) to the external environment (95).

9. The methane oxidation device (100) of any preceding claim, wherein the methane oxidation unit (115) is dome-shaped.

10. The methane oxidation device (100) of any preceding claim, wherein the first portion (105a) of at least one hollow member (105) is in fluid communication with the external environment (95) via a permeable mesh (125).

11. The methane oxidation device (100) of any preceding claim, wherein the at least one hollow member (105) comprises an unclogging member configured to unclog accumulated matter from at least a portion of the at least one hollow member (105).

12. The methane oxidation device (100) of any preceding claim, wherein the methane oxidation device (100) comprises a methane dilution unit configured to dilute the concentration of methane.

13. The methane oxidation device (100) of any preceding claim, wherein the methane oxidation unit (115) comprises a cooling unit configured to lower the temperature within the methane oxidation unit (115).

14. The methane oxidation device (100) of any preceding claim, wherein the at least one hollow member (105) comprises a buoyant member (155) configured to float inside the digestive cavity (55) associated with the animal (50).

15. An installation gun for installing the methane oxidation device (100) of any preceding claim; the installation gun comprising;
a methane oxidation device holder configured to hold a methane oxidation device (100) according to any preceding claim;
an insertion unit configured to insert a portion of a methane oxidation device (100) held on the methane oxidation device holder into a digestive cavity (55) associated with an animal (50); and
an actuation member configured for user actuation of the insertion unit; and
**characterised by** further comprising:
an ignition member configured to produce a spark.

## Patentansprüche

1. Eine Methanoxidationsvorrichtung (100) zur Oxidation von Methan aus einer einem Tier (50) zugehörigen Verdauungshöhle (55), wobei die Vorrichtung (100) Folgendes beinhaltet:
mindestens ein hohles Element (105), das einen ersten Abschnitt (105a) und einen zweiten Abschnitt (105b) beinhaltet;
ein Stechelement (110), das konfiguriert ist, um einen Kanal (60a) in die Verdauungshöhle (55) zu stechen, wobei sich das Stechelement (110) an dem ersten Abschnitt (105a) des mindestens einen hohlen Elements (105) befindet, sodass der erste Abschnitt (105a) konfiguriert ist, um in Fluidverbindung mit der Verdauungshöhle (55) angeordnet zu werden; und
eine Methanoxidationseinheit (115) zum Oxidieren von Methan, die konfiguriert ist, um sich an dem zweiten Abschnitt (105b) des mindestens einen hohlen Elements (105) zu befinden;
wobei die Methanoxidationseinheit (115) Folgendes beinhaltet:
einen Einlassabschnitt (120) in Fluidverbindung mit der externen Umgebung (95), der an dem mindestens einen hohlen Element (105) montiert ist; und
einen Auslassabschnitt (130) in Fluidverbindung mit der externen Umgebung (95),
sodass Methan aus der dem Tier (50) zugehörigen Verdauungshöhle (55) vor der Freisetzung in die externe Umgebung (95) in der Oxidationseinheit (115) oxidiert wird.

2. Methanoxidationsvorrichtung (100) gemäß Anspruch 1, wobei sich die Methanoxidationseinheit (115) zwischen dem mindestens einen hohlen Element (105) und der externen Umgebung (95) befindet.

3. Methanoxidationsvorrichtung (100) gemäß Anspruch 1 oder Anspruch 2, wobei die Methanoxidationseinheit (115) mindestens ein katalytisches Material (140) beinhaltet; und wobei vorzugsweise
sich das mindestens eine katalytische Material (140) zwischen dem Einlassabschnitt (120) und dem Auslassabschnitt (130) befindet und mindestens ein Anteil des mindestens einen katalytischen Materials (140) in einer durchlässigen Konfiguration angeordnet ist, um einen Fluidfluss von dem Einlassabschnitt (120) zu dem Auslassabschnitt (130) zu ermöglichen.

4. Methanoxidationsvorrichtung (100) gemäß einem vorhergehenden Anspruch, wobei die Methanoxidationsvorrichtung (115) ein Sicherungselement (150) beinhaltet, das konfiguriert ist, um die Methanoxidationseinheit (115) entfernbar an dem Tier (50) zu sichern; und wobei vorzugsweise
sich das Sicherungselement (150) an dem ersten Abschnitt (105a) des mindestens einen hohlen Elements (105) befindet und einen zusammenfaltbaren Abschnitt (150b) beinhaltet, wobei das Zurückziehen der Methanoxidationsvorrichtung (115) durch den Kanal (60a) in die Höhle (55) des Tiers (50) den ersten Abschnitt (105a) zu dem zweiten Abschnitt (105b) hin zieht, um den zusammenfaltbaren Abschnitt (150b) zusammenzufalten, wobei der zusammenfaltbare Abschnitt (150b) konfiguriert ist, um sich in Bezug auf eine Längsachse (L) des mindestens einen hohlen Elements (105) nach außen zu spreizen, wenn er zusammengefaltet wird.

5. Methanoxidationsvorrichtung (100) gemäß einem vorhergehenden Anspruch, wobei das mindestens eine hohle Element (105) ein Ventil (145) beinhaltet, das sich in dem zweiten Abschnitt (105b) befindet und konfiguriert ist, um zu verhindern, dass ein Fluidfluss aus dem zweiten Abschnitt (105b) des mindestens einen hohlen Elements (105) austritt.

6. Methanoxidationsvorrichtung (100) gemäß einem vorhergehenden Anspruch, wobei der Auslassabschnitt (130) eine Vielzahl von Lüftungsöffnungen (135) beinhaltet, die sich an einer externen Oberfläche der Methanoxidationseinheit (115) befinden.

7. Methanoxidationsvorrichtung (100) gemäß einem vorhergehenden Anspruch, wobei die Methanoxidationsvorrichtung (100) einen Sensor (200) beinhaltet; und wobei vorzugsweise
die Methanoxidationsvorrichtung (100) eine Datenübertragungseinheit beinhaltet, die konfiguriert ist, um Daten von dem Sensor (200) an eine externe Vorrichtung zu übertragen; und/oder wobei vorzugsweise
das mindestens eine hohle Element (105) einen Flussregler (180) beinhaltet, der konfiguriert ist, um einen Fluidfluss in die Methanoxidationseinheit (115) zu verhindern, wenn der Sensor (200) detektiert, dass eine Eigenschaft der Vorrichtung (100) bei einem vorgegeben Schwellenwert liegt.

8. Methanoxidationsvorrichtung (100) gemäß einem vorhergehenden Anspruch, wobei die Methanoxidationseinheit (115) eine Wärmebarriere (160) beinhaltet, die konfiguriert ist, um eine Wärmeübertragung von der Methanoxidationseinheit (115) an die externe Umgebung (95) zu verhindern.

9. Methanoxidationsvorrichtung (100) gemäß einem vorhergehenden Anspruch, wobei die Methanoxidationseinheit (115) kuppelförmig ist.

10. Methanoxidationsvorrichtung (100) gemäß einem vorhergehenden Anspruch, wobei der erste Abschnitt (105a) mindestens eines hohlen Elements (105) über ein durchlässiges Sieb (125) in Fluidverbindung mit der externen Umgebung (95) steht.

11. Methanoxidationsvorrichtung (100) gemäß einem vorhergehenden Anspruch, wobei das mindestens eine hohle Element (105) ein Verstopfung beseitigendes Element beinhaltet, das konfiguriert ist, um eine Verstopfung durch angesammelte Materie von mindestens einem Abschnitt des mindestens einen hohlen Elements (105) zu beseitigen.

12. Methanoxidationsvorrichtung (100) gemäß einem vorhergehenden Anspruch, wobei die Methanoxidationsvorrichtung (100) eine Methanverdünnungseinheit beinhaltet, die konfiguriert ist, um die Konzentration von Methan zu verdünnen.

13. Methanoxidationsvorrichtung (100) gemäß einem vorhergehenden Anspruch, wobei die Methanoxidationseinheit (115) eine Kühleinheit beinhaltet, die konfiguriert ist, um die Temperatur innerhalb der Methanoxidationseinheit (115) zu senken.

14. Methanoxidationsvorrichtung (100) gemäß einem vorhergehenden Anspruch, wobei das mindestens eine hohle Element (105) ein Auftriebselement (155) beinhaltet, das konfiguriert ist, um innerhalb der dem Tier (50) zugehörigen Verdauungshöhle (55) zu schwimmen.

15. Eine Installationspistole zum Installieren der Methanoxidationsvorrichtung (100) gemäß einem vorhergehenden Anspruch; wobei die Installationspistole Folgendes beinhaltet:
einen Methanoxidationsvorrichtungshalter, der konfiguriert ist, um eine Methanoxidationsvorrichtung (100) gemäß einem vorhergehenden Anspruch zu halten;
eine Einführeinheit, die konfiguriert ist, um einen Abschnitt einer an dem Methanoxidationsvorrichtungshalter gehaltenen Methanoxidationsvorrichtung (100) in eine einem Tier (50) zugehörige Verdauungshöhle (55) einzuführen; und
ein Betätigungselement, das zur Benutzerbetätigung der Einführeinheit konfiguriert ist; und
**dadurch gekennzeichnet, dass** sie ferner Folgendes beinhaltet:
ein Zündelement, das konfiguriert ist, um einen Funken zu produzieren.

## Revendications

1. Un dispositif d'oxydation de méthane (100) pour l'oxydation de méthane issu d'une cavité digestive (55) associée à un animal (50), le dispositif (100) comprenant :
au moins un élément creux (105) comprenant une première portion (105a) et une deuxième portion (105b) ;
un élément de perçage (110) configuré pour percer un canal (60a) dans ladite cavité digestive (55), l'élément de perçage (110) étant situé au niveau de la première portion (105a) de l'au moins un élément creux (105) de telle sorte que la première portion (105a) est configurée pour être agencée en communication fluidique avec ladite cavité digestive (55) ; et
une unité d'oxydation de méthane (115) pour oxyder du méthane et configurée pour être située au niveau de la deuxième portion (105b) de l'au moins un élément creux (105) ;
dans lequel l'unité d'oxydation de méthane (115) comprend :
une portion d'entrée (120) en communication fluidique avec l'environnement externe (95) et montée sur l'au moins un élément creux (105) ; et
une portion de sortie (130) en communication fluidique avec l'environnement externe (95),
de sorte que le méthane issu de ladite cavité digestive (55) associée audit animal (50) est oxydé dans l'unité d'oxydation (115) avant d'être libéré dans l'environnement externe (95).

2. Le dispositif d'oxydation de méthane (100) de la revendication 1, dans lequel l'unité d'oxydation de méthane (115) est située entre l'au moins un élément creux (105) et l'environnement externe (95).

3. Le dispositif d'oxydation de méthane (100) de la revendication 1 ou de la revendication 2, dans lequel l'unité d'oxydation de méthane (115) comprend au moins un matériau catalytique (140) ; et de préférence
dans lequel l'au moins un matériau catalytique (140) est situé entre la portion d'entrée (120) et la portion de sortie (130) et au moins une portion de l'au moins un matériau catalytique (140) est agencée en une configuration perméable de façon à permettre un écoulement de fluide de la portion d'entrée (120) à la portion de sortie (130).

4. Le dispositif d'oxydation de méthane (100) de n'importe quelle revendication précédente, le dispositif d'oxydation de méthane (115) comprenant un élément de retenue (150) configuré pour retenir de manière amovible l'unité d'oxydation de méthane (115) sur ledit animal (50) ; et de préférence
dans lequel l'élément de retenue (150) est situé au niveau de la première portion (105a) de l'au moins un élément creux (105) et comprend une portion repliable (150b) dans lequel la rétraction du dispositif d'oxydation de méthane (115) à travers le canal (60a) dans la cavité (55) de l'animal (50) attire la première portion (105a) vers la deuxième portion (105b) pour replier la portion repliable (150b), la portion repliable (150b) étant configurée pour s'évaser vers l'extérieur par rapport à un axe longitudinal (L) de l'au moins un élément creux (105) à mesure qu'elle se replie.

5. Le dispositif d'oxydation de méthane (100) de n'importe quelle revendication précédente, dans lequel l'au moins un élément creux (105) comprend un clapet (145) situé dans la deuxième portion (105b) et configuré pour empêcher un écoulement de fluide de sortir de la deuxième portion (105b) de l'au moins un élément creux (105).

6. Le dispositif d'oxydation de méthane (100) de n'importe quelle revendication précédente, dans lequel la portion de sortie (130) comprend une pluralité d'évents (135) situés sur une surface externe de l'unité d'oxydation de méthane (115).

7. Le dispositif d'oxydation de méthane (100) de n'importe quelle revendication précédente, le dispositif d'oxydation de méthane (100) comprenant un capteur (200) ; et de préférence
le dispositif d'oxydation de méthane (100) comprenant une unité de transfert de données configurée pour transférer des données du capteur (200) à un dispositif externe ; et/ou de préférence
dans lequel l'au moins un élément creux (105) comprend un régulateur d'écoulement (180) configuré pour empêcher un écoulement de fluide dans l'unité d'oxydation de méthane (115) lorsque le capteur (200) détecte qu'une caractéristique du dispositif (100) est à un seuil prédéterminé.

8. Le dispositif d'oxydation de méthane (100) de n'importe quelle revendication précédente, dans lequel l'unité d'oxydation de méthane (115) comprend une barrière thermique (160) configurée pour empêcher un transfert de chaleur de l'unité d'oxydation de méthane (115) à l'environnement externe (95).

9. Le dispositif d'oxydation de méthane (100) de n'importe quelle revendication précédente, dans lequel l'unité d'oxydation de méthane (115) est en forme de dôme.

10. Le dispositif d'oxydation de méthane (100) de n'importe quelle revendication précédente, dans lequel la première portion (105a) d'au moins un élément creux (105) est en communication fluidique avec l'environnement externe (95) par l'intermédiaire d'un maillage perméable (125).

11. Le dispositif d'oxydation de méthane (100) de n'importe quelle revendication précédente, dans lequel l'au moins un élément creux (105) comprend un élément de désobstruction configuré pour désobstruer la matière accumulée d'au moins une portion de l'au moins un élément creux (105).

12. Le dispositif d'oxydation de méthane (100) de n'importe quelle revendication précédente, le dispositif d'oxydation de méthane (100) comprenant une unité de dilution de méthane configurée pour diluer la concentration de méthane.

13. Le dispositif d'oxydation de méthane (100) de n'importe quelle revendication précédente, dans lequel l'unité d'oxydation de méthane (115) comprend une unité de refroidissement configurée pour abaisser la température à l'intérieur de l'unité d'oxydation de méthane (115).

14. Le dispositif d'oxydation de méthane (100) de n'importe quelle revendication précédente, dans lequel l'au moins un élément creux (105) comprend un élément flottant (155) configuré pour flotter à l'intérieur de la cavité digestive (55) associée à l'animal (50).

15. Un pistolet d'installation pour installer le dispositif d'oxydation de méthane (100) de n'importe quelle revendication précédente ; le pistolet d'installation comprenant :
un organe de tenue de dispositif d'oxydation de méthane configuré pour tenir un dispositif d'oxydation de méthane (100) selon n'importe quelle revendication précédente ;
une unité d'insertion configurée pour insérer une portion d'un dispositif d'oxydation de méthane (100) tenu sur l'organe de tenue de dispositif d'oxydation de méthane dans une cavité digestive (55) associée à un animal (50) ; et
un élément d'actionnement configuré pour l'actionnement par un utilisateur de l'unité d'insertion ;
et
**caractérisé en ce qu'**il comprend en outre :
un élément d'allumage configuré pour produire une étincelle.
